# EUROPÄISCHE PATENTSCHRIFT

(11) **EP 1 204 475 B1**
(45) Veröffentlichungstag und Bekanntmachung des Hinweises auf die Patenterteilung: **23.06.2004**
(21) Anmeldenummer: 00943874.8
(22) Anmeldetag: 23.06.2000
(51) Int. Cl.: B01J 35/00, B01J 31/18, B01J 31/16, C07D 309/04

(54) **IMMOBILISIERTER PHOTOKATALYSATOR**
IMMOBILIZED PHOTOCATALYST
PHOTOCATALYSEUR IMMOBILISE

(30) Priorität: 25.06.1999 DE 19929053
(43) Veröffentlichungstag der Anmeldung: 15.05.2002
(73) Patentinhaber: ProSys Gesellschaft für produktionsintegrierte Umweltsystemtechnologien und -management mbH, 28359 Bremen (DE)
(72) Erfinder: SCHNEIDER V.D. FECHT, Gerhard, D-28203 Bremen (DE); WÖHRLE, Dieter, D-28359 Bremen (DE); GERDES, Robert, D-28259 Bremen (DE)
(74) Vertreter: Stilkenböhmer, Uwe Michael, Dr.
(86) Internationale Anmeldenummer: PCT/EP2000/005832
(87) Internationale Veröffentlichungsnummer: WO 2001/000322

(56) Entgegenhaltungen:
- EP-A- 0 168 994
- WO-A-91/01806
- US-A- 4 800 188
- US-A- 4 806 514

## Beschreibung

Die Erfindung betrifft immobilisierte Photokatalysatoren, ein Verfahren zur Photooxidation von Substanzen und die Verwendung des Photokatalysators.

Ein äußerst reaktives Mittel, um Moleküle zu oxidieren, ist Singulett-Sauerstoff, der photochemisch erzeugt werden kann. Sauerstoff liegt im Grundzustand im Triplettzustand vor und kann durch Zufuhr einer vorbestimmten Energiemenge in den Singulettzustand überführt werden. Am einfachsten erzeugt man den Singulett-Sauerstoff durch Einstrahlung von sichtbarem Licht, insbesondere Tageslicht, in Gegenwart eines Photosensibilisators, der die entsprechende Energiemenge auf den Triplettsauerstoff übertragen kann. Hierzu sind viele Verbindungen bekannt; dies sind Farbstoffe, wie Bengalrosa, Kongorot, Methylenblau, Kristallviolett oder Alizarin. Diese Photosensibilisatoren werden während der Reaktion verbraucht und eignen sich deshalb weniger für Photooxidationen mit Luftsauerstoff.

Photochemisch erzeugter Singulett-Sauerstoff wird vorteilhaft für Oxidationen anstelle von Ozon eingesetzt, da dies sowohl Kostenvorteile bringt als auch für die Umwelt günstig ist. Ein Nachteil besteht allerdings darin, daß die als Photosensibilisatoren verwendeten Farbstoffe nach der Reaktion nicht ohne weiteres abgetrennt oder entfernt werden können.

Um dieses Problem zu lösen, wurde bereits vorgeschlagen, Farbstoffe, die als Photosensibilisatoren verwendet werden, an Träger zu binden. So beschreibt beispielsweise US-PS 4008136 ein Verfahren zur Behandlung von Abwasser, bei dem Farbstoffe, wie insbesondere Bengalrosa, an einen Ionenaustauscher gebunden werden und dann zur Abwasseroxidation verwendet werden. Ein Nachteil dieses Verfahrens besteht darin, daß Bengalrosa zwar ein wirkungsvoller Photosensibilisator ist, allerdings nur eine geringe Stabilität aufweist. Es muß daher während des Verfahrens ständig nachdosiert werden.

Aufgabe der Erfindung war es daher, einen immobilisierten Photokatalysator bereitzustellen, der bei Bestrahlung mit sichtbarem Licht Sauerstoff in die Singulettform überführen kann, leicht nach der Reaktion wieder abgetrennt werden kann und eine hohe Aktivität und Stabilität aufweist.

Diese Aufgabe wird gelöst mit einem Photokatalysator, der einen photokatalytisch aktiven Bestandteil immobilisiert an einem Ionenaustauscherharz umfaßt, wobei der photokatalytisch aktive Bestandteil ausgewählt ist aus Verbindungen der Formeln I und II und Kombinationen davon 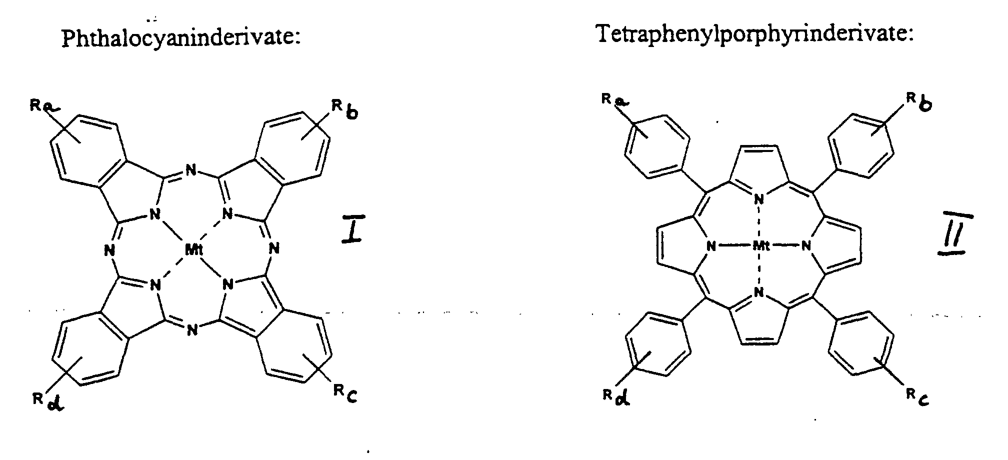 worin Mt ein Metall ist ausgewählt aus Aluminium, Silicium, Gallium und/oder Germanium und alle Reste R entweder jeweils unabhängig aus anionischen Gruppen oder aus kationischen Gruppen ausgewählt sind und die Indices a, b, c, und d jeweils unabhängig 0 oder ganze Zahlen von 1 bis 4 bedeuten, mit dem Vorbehalt, daß mindestens einer der Indices a, b, c und d eine andere Bedeutung als 0 hat.

Überraschenderweise wurde gefunden, daß die Immobilisierung eines Metall-Phthalocyaninderivats und/oder Metall-Tetraphenylporphyrinderivats an einem Ionenaustauscherharz als Träger einen überaus beständigen und reaktiven Photosensibilisator erzeugt, der eine so hohe Aktivität aufweist, daß er sogar bei geringer Lichteinstrahlung, z.B.diffusem sichtbaren Licht, Photooxidationsprozesse katalysieren kann. Die Anregungswellenlänge für die erfindungsgemäßen Photokatalysatoren liegt in einem Bereich von 400 bis 800 nm, daher können sie das Spektrum des sichtbaren Lichts ausnutzen. Außerdem ist die Stabilität der erfindungsgemäßen Photokatalysatoren hoch, so daß sie ohne merklichen Abbau und mit unveränderter Aktivität über längere Zeiträume, z.B. 100 Oxidationszyklen und mehr, eingesetzt werden können. Im Gegensatz dazu werden die obenerwähnten bekannten Photosensibilisatoren bei einem Zyklus abgebaut und müssen daher in größerer Menge verwendet und immer wieder ergänzt werden.

Erfindungsgemäß wird eine Kombination aus einem Ionenaustauscherharz mit einem Metall-Phthalocyaninderivat und/oder Metall-Tetraphenylporphyrinderivat eingesetzt. Als Ionenaustauscherharz kann jedes handelsübliche Kationen- oder Anionenaustauscherharz mit basischen bzw. sauren Gruppen verwendet werden. Ionenaustauscher sind dem Fachmann wohlbekannt und bedürfen hier keiner nähere Erläuterung. Bevorzugt werden für die erfindungsgemäßen Photokatalysatoren Anionenaustauscher verwendet. Als besonders geeignet haben sich Anionenaustauscher mit Trimethylammoniumgruppen erwiesen, die unter der Bezeichnung Amberlite IRA 400 im Handel erhältlich sind.

An basische bzw. kationische oder saure bzw. anionische Gruppen des Harzes binden negativ bzw. positiv geladene Gruppen des Metall-Phthalocyanin- bzw. Tetraphenylporphyrinkomplexes und haften auf der Oberfläche des Austauscherharzes aufgrund von Coloumb-Wechselwirkungen. Diese Wechselwirkungen sind so stark und stabil, daß die Komplexe weder in wässriger noch in organischer Phase und auch nicht bei unterschiedlichen pH-Werten herausgelöst werden. Sie sind daher sehr beständig und können für vielfältige Reaktionen eingesetzt werden.

Als aktiver Bestandteil kommen erfindungsgemäß Metall-Phthalocyaninderivate der Formel I und/oder Metall-Tetraphenylporphyrinderivate der Formel II zum Einsatz. Der Ausdruck "Phthalocyaninderivat" umfaßt in dieser Beschreibung und den Ansprüchen Phthalocyaninverbindungen, die an den aromatischen Ringen mit ein oder mehreren Substituenten in beliebiger Position in an sich bekannter Weise substituiert sind. Ebenso umfaßt der Ausdruck "Tetraphenylporphyrinderivat" Tetraphenylporphyrinverbindungen, die an den aromatischen Ringen in beliebiger Position einfach oder mehrfach substituiert sind.

Für die Bindung an das lonenaustauscherharz muß mindestens eine funktionelle Gruppe an dem Phthalocyanin- bzw. Porphyrinkomplex verfügbar sein. Geeignet ist hier jeder Substituent, der eine Bindung an ein Ionenaustauscherharz eingehen kann. Der Substituent bzw. die Substituenten R sind außen an den Komplexverbindungen gebunden. Obwohl eine funktionelle Gruppe zur Bindung an das Ionenaustauscherharz ausreicht, ist es bevorzugt mindestens 2 und besonders bevorzugt 4 Substituenten, die bevorzugt an verschiedenen Phenylringen gebunden sind, zu verwenden.

Als Substituenten zur Bindung an das Ionenaustauscherharz werden zur Bindung an Anionenaustauscherharze saure bzw. anionische Gruppen und zur Bindung an Kationenaustauscherharze basische bzw. kationische Gruppen verwendet. Geeignet sind alle Substituenten, die einerseits eine Bindung an das Ionenaustauscherharz vermitteln können und andererseits die Funktion des Phthtalocyanin- oder Tetraphenylporphyrinderivats nicht stören. Bevorzugt werden als saure Gruppen phenolische Gruppen, Hydroxygruppen, Halogene oder Säuregruppen, wie Carboxyl-, Carbonsäure- und Sulfonsäurereste eingesetzt, während als bevorzugte basische Gruppen, Amino-, Trialkylammonium-, Trialkylphosphonium-, Triarylphosponium- oder Alkyl- oder Arylpyridiniumgruppen zum Einsatz kommen, wobei die Alkylreste z.B. 1 bis 18 C-Atome aufweisen können und bevorzugt kurzkettige Alkylreste mit 1 bis 6, besonders bevorzugt 1 bis 2 Kohlenstoffatomen sind und die Arylreste bevorzugt 6 bis 12 C-Atome aufweisen. Ein oder mehrere Sulfonsäure- und/oder Carboxylgruppen sind als saure Substituenten bevorzugt. Besonders bevorzugt sind die Phthalocyanin- und Tetraphenylporphyrinderivate bei denen R jeweils ein Sulfonsäure- oder Carboxylrest ist und a, b, c und d jeweils 1 sind. Als basische Gruppen sind Trimethylammoniumgruppen besonders bevorzugt.

Das Zentralatom von Phthalocyanin oder Tetraphenylporphyrin ist ein Metall aus Aluminium, Silicium, Gallium und/oder Germanium. Für die erfindungsgemäßem Photokatalysatoren werden solche Verbindungen bevorzugt, bei denen das Zentralatom, Al(III), Ga(III), Si(IV) und/oder Ge(IV) ist.

In einer bevorzugten Ausführungsform der Erfindung wird als Photokatalysator eine Mischung verschiedener Metall-Phthalocyanin- und/oder Tetraphenylporphyrinderivate eingesetzt, wobei entweder an einem Träger verschiedene Verbindungen immobilisiert sein können, oder aber mehrere Träger, die jeweils unterschiedliche Sensibilisatoren tragen, verwendet werden können. Mit einer derartigen Mischung von Verbindungen, die verschiedene Anteile des sichtbaren Lichtspektrums verwerten, kann die Spektrenausnutzung optimiert werden.

Es wurde festgestellt, daß bei Verwendung der erfindungsgemäß eingesetzten Phthalocyanin- und/oder Tetraphenylporphyrinkomplexe bei der Photooxidation eine höhere Reaktiongeschwindigkeit erhalten wird, als mit bisher bekannten und verwendeten Photosensibilisatorträgersystemen. Darüberhinaus sind die erfindungsgemäßen Photokatalysatoren ziemlich unempfindlich gegenüber Änderungen des pH-Wertes, der Temperatur und gegenüber Lösungsmittelvariationen. Daher ist mit diesen Katalysatoren eine optimale Prozeßsteuerung möglich.

Die erfindungsgemäßen Photokatalysatoren können in einfacher Weise hergestellt werden, indem die gewünschte Menge an Phthalocyanin- und/oder Tetraphenylporphyrinderivat an ein Ionenaustauscherharz gebunden wird. Die Bindung erfolgt quantitativ und schnell über die ionischen Gruppen. Zur Herstellung muß daher einfach nur der Ionenaustauscher mit den Derivaten vermischt werden. In einer bevorzugten Ausführungsform werden zur Herstellung der an Ionenaustauscher gebundenen Photosensibilisatoren das ausgewählte Derivat bzw. die Derivate in definierter Menge einzeln gemischt und mit einer definierten Menge Anionen- bzw. Kationenaustauscher in Wasser, das bevorzugt etwas erwärmt wurde, z.B. auf eine Temperatur zwischen 40 und 80, bevorzugt 50 und 70°C, versetzt. Die Mischung wird solange gerührt, bis die Derivate gebunden sind. Dies ist in der Regel nach einem Zeitraum von ca. 6 bis 12 Stunden der Fall. Die eingesetzten Komponenten, Ionenaustauscherharz und Phthalocyanin- bzw. Tetraphenylporphyrinderivat müssen gegenionisch sein, d.h. wenn ein Anionenaustauscher eingesetzt wird, muß das Phthalocyanin- oder Tetraphenylporphyrinderivat saure Gruppen aufweisen, während es dann, wenn ein Kationenaustauscher eingesetzt wird, basische Gruppen aufweisen muß. Die Bindung erfolgt in jedem Falle quantitativ. Daher kann die gewünschte Konzentration an Photosensibilisator sehr einfach durch die Einstellung der eingesetzten Mengen der Komponenten eingestellt werden.

Die erfindungsgemäßen immobilisierten Photokatalysatoren können für jede Artvon Photooxidation eingesetzt werden, bei der Singulett-Sauerstoff als Oxidationsmittel verwendet werden soll. Sie sind zum Beispiel geeignet zur Oxidation von komplexen organischen Molekülen, wie Steroiden, Phenolen, Chlorphenolen, Terpenen oder zur Oxidation von schwefelhaltigen Gruppen, wie Thiol- und Sulfidgruppen.

Da die erfindungsgemäßen Photokatalysatoren nach der Reaktion sehr leicht von dem Reaktionsansatz abgetrennt werden können und auch keine unerwünschten Rückstände in den oxidierten Substanzen zurückbleiben, eignen sie sich besonders gut zur Oxidation von Substanzen, die zur Herstellung von Lebensmitteln, Bedarfsgegenständen, Kosmetika, Arzneimitteln und sonstigen Mitteln, die mit Lebensmitteln oder dem menschlichen Körper in Kontakt kommen, verwendet werden.

Andererseits sind die erfindungsgemäßen Photokatalysatoren gut geeignet zur Abwasserreinigung. Die Umwelt wird durch die Katalysatoren nicht belastet, da diese nach der Behandlung wieder entfernt werden, im Gegensatz zu chemischen Oxidationsmitteln, deren Rückstände im Abwasser zurückbleiben.

Ein weiteres Verfahren der Erfindung ist daher ein Verfahren zur Oxidation von Verbindungen unter Verwendung von Singulett-Sauerstoff als Oxidationsmittel, wobei der Singulett-Sauerstoff erzeugt wird, indem die zu oxidierende Verbindung unter Einstrahlung von sichtbarem Licht und in Gegenwart von Sauerstoff mit einem Photokatalysator, wie oben definiert, in Kontakt gebracht wird.

Besonders geeignet ist dieses Verfahren zur Oxidation von biologischem Material, insbesondere in Abwasser. Ein weiterer Gegenstand der Erfindung ist daher die Verwendung des erfindungsgemäßen immobilisierten Photokatalysators zur Behandlung von Abwasser.

Es wurde festgestellt, daß die erfindungsgemäßen Photokatalysatoren für viele Photooxidationen erfolgreich eingesetzt werden können. So können die erfindungsgemäßen Katalysatoren fürdie Photooxidation so unterschiedlicher Substanzen wie Thiole, Sulfide, Chlorphenole, Terpene, Steroide, Cyclopentadien und β-Citronellol verwendet werden.

Als vorteilhaft hat sich unter anderem die Verwendung des erfindungsgemäßen Photokatalysators zur Herstellung von Rosenoxid erwiesen. Rosenoxid ist ein Grundstoff der Parfümindustrie und wird aus β-Citronellol hergestellt.

Ein weiterer Gegenstand der Erfindung ist daher die Verwendung des immobilisierten Photokatalysators zur Herstellung von Rosenoxid durch Photooxidation von β-Citronellol.

Die Erfindung wird durch die folgenden Beispiele erläutert.

### Beispiel 1

Es wurde ein erfindungsgemäßer Photosensibilisator hergestellt. Dazu wurde das Anionenaustauscherharz Amberlite IRA 400 in Wasser gegeben und eine Mischung aus Phthalocyanintetrasulfonat und Tetraphenylporphyrintetrasulfonat in einem Verhältnis von 1:1 zugegeben. Das Wasser hatte eine Temperatur von 60°C. Die Mischung wurde 12 Stunden lang gerührt. Danach waren die Phthalocyanin- und Tetraphenylporphyrinderivate quantitativ an dem Anionenaustauscherharz gebunden. Der Photokatalysator konnte für verschiedene Oxidationen eingesetzt werden.

### Beispiel 2

Mit dem in Beispiel 1 hergestellten Photokatalysator wurden Thiole und Phenole oxidiert. Die Reaktionen sind dem folgenden Schema zu entnehmen.

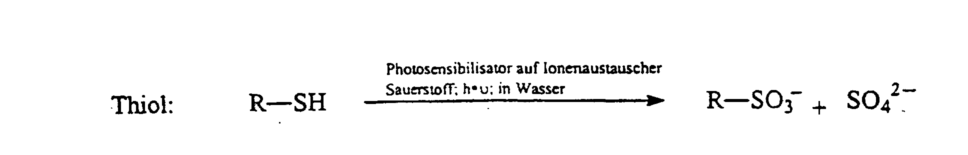

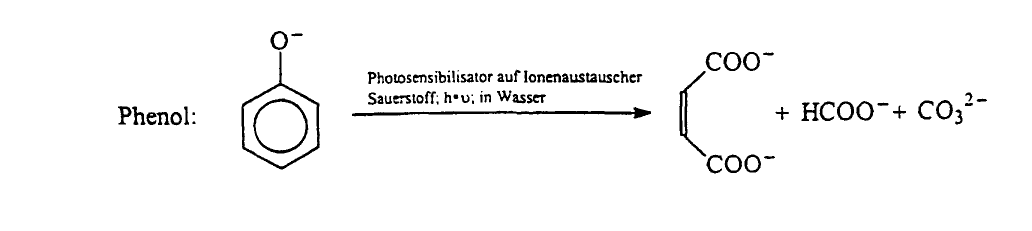

In einer wäßrig-alkalischen Lösung (pH 9 bis 13, 50 ml) wurde der an Ionenaustauscher gebundene Photosensibilisator suspendiert. Dann wurde eine definierte Menge an Thiol oder Phenol zugegeben, wobei ein Konzentrationsverhältnis von Photosensibilisator zu Substrat von 1:1500 eingestellt wurde. Anschließend wurde die Lösung mit Sauerstoff gesättigt und mit sichtbarem Licht bestrahlt. Das Thiol wurde bei dieser Reaktion zum Alkylsulfonat, bzw. Sulfat oxidiert, das Phenol zu Maleinat, Formiat und Carbonat. Da Thiole und Phenole in kontaminierten Abwässern enthalten sind, können diese Reaktionen zur Klärung kontaminierter Abwässer eingesetzt werden.

### Beispiel 3

Dererfindungsgemäße Photokatalysator wurde zur Photooxidation von β-Citronellol eingesetzt, wie dem folgenden Reaktionsschema zu entnehmen ist.

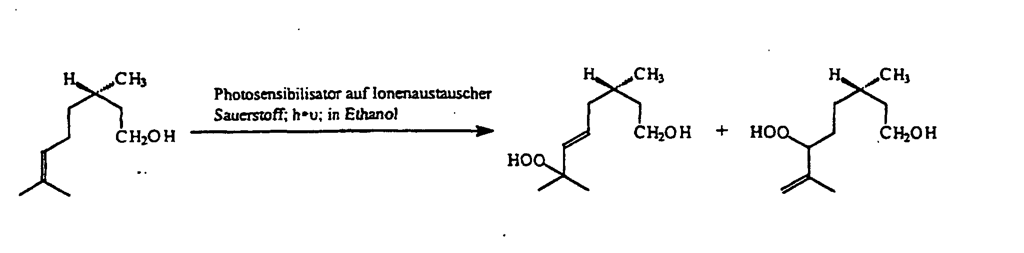

Dazu wurde der in Beispiel 1 hergestellte Photosensibilisator in 50 ml Methanol suspendiert und dann eine definierte Menge an β-Citronellol zugegeben. Die Lösung wurde mit Sauerstoff gesättigt und mit sichtbarem Licht bestrahlt. β-Citronellol wurde bei dieser Reaktion zu einem Gemisch von cis- und trans-Rosenoxid oxidiert, die beide als Grundstoff in der Parfümindustrie Verwendung finden.

## Patentansprüche

1. Photokatalysator, der einen photokatalytisch aktiven Bestandteil immobilisiert an einem lonenaustauscherharz umfaßt, wobei der photokatalytisch aktive Bestandteil ausgewählt ist aus Verbindungen der Formeln I und II und Kombinationen davon 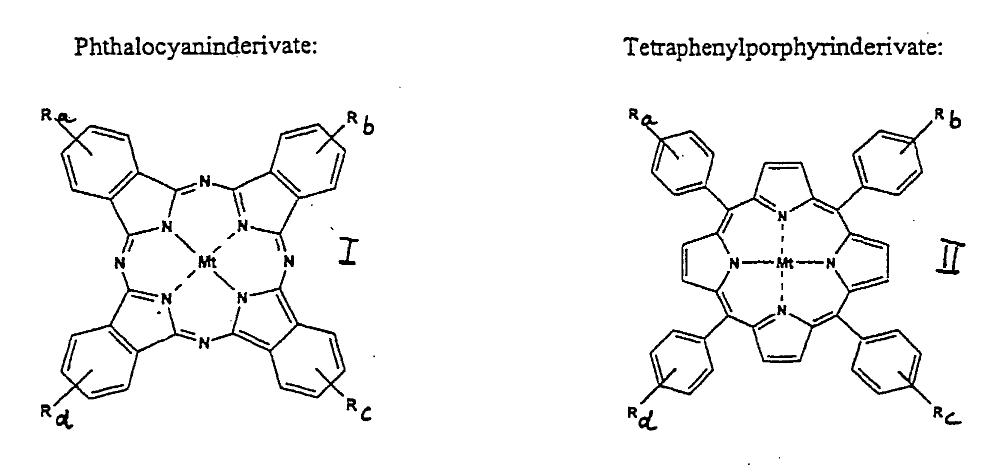 worin Mt ein Metall ist ausgewählt aus Aluminium, Silicium, Gallium und/oder Germanium und alle Reste R entweder jeweils unabhängig ausgewählt sind aus anionischen Gruppen oder aus kationischen Gruppen und die Indices a, b, c, und d jeweils unabhängig 0 oder ganze Zahlen von 1 bis 4 bedeuten, mit dem Vorbehalt, daß mindestens einer der Indices a, b, c und d eine andere Bedeutung als 0 hat.

2. Photokatalysator nach Anspruch 1,
**dadurch gekennzeichnet, daß** der Träger ein Anionenaustauscherharz ist.

3. Photokatalysator nach Anspruch 1 oder Anspruch 2,
**dadurch gekennzeichnet, daß** der Substituent R ein Halogen, OH, eine phenolische Gruppe, -COOH, -SO₃H, eine Carbonsäuregruppe oder Kombinationen davon sind.

4. Photokatalysator nach Anspruch 1,
**dadurch gekennzeichnet, daß** der Substituent R ein Amino-, Trialkylammonium-, Trialkylphosphonium-, Triarylphosphonium-, Alkyl- oder Arylpyridiniumrest, oder Kombinationen davon sind, wobei die Alkylreste jeweils 1 bis 18 Kohlenstoffatome aufweisen können und die Arylreste 6 bis 12 Kohlenstoffatome aufweisen können.

5. Photokatalysator nach einem der vorhergehenden Ansprüche, **dadurch gekennzeichnet, daß** der aktive Bestandteil ein Metall-Phthalocyaninund/oder Tetraphenylporphyrinderivat ist, worin R ein Sulfonsäure- oder Carboxylrest ist und a, b, c und d jeweils 1 sind.

6. Photokatalysator nach einem der vorhergehenden Ansprüche, **dadurch gekennzeichnet, daß** der aktive Bestandteil Phthalocyanin- oder Tetraphenylporphyrintetrasulfonat oder eine Mischung davon ist.

7. Photokatalysator nach einem der vorhergehenden Ansprüche,
**dadurch gekennzeichnet, daß** das Metall des Phthalocyanins oder Tetraphenylporphyrins Aluminium(III), Silicium(IV), Gallium(III) und/oder Germanium(IV) ist.

8. Verfahren zur Photooxidation unter Verwendung von Singulett-Sauerstoff als Oxidationsmittel, wobei der Singulett-Sauerstoff erzeugt wird, indem man die zu oxidierende Verbindung unter Einstrahlung von Licht und in Gegenwart von Sauerstoff mit einem Photokatalysator nach einem der Ansprüche 1 bis 7 in Kontakt bringt.

9. Verfahren nach Anspruch 8, **dadurch gekennzeichnet, daß** mit Licht einer Wellenlänge von 400 bis 800 nm bestrahlt wird.

10. Verfahren nach Anspruch 8 oder 9, **dadurch gekennzeichnet, daß** mit sichtbarem Licht oder Sonnenlicht bestrahlt wird.

11. Verfahren zur Photooxidation von biologischem Material, **dadurch gekennzeichnet, daß** man das biologische Material mit einem immobilisiertem Photokatalysator nach einem der Ansprüche 1 bis 7 unter Einstrahlung von sichtbarem Licht und in Gegenwart von Sauerstoff in Kontakt bringt.

12. Verfahren nach einem der Ansprüche 8 bis 11,
**dadurch gekennzeichnet, daß** man eine Mischung von Photokatalysatoren nach einem der Ansprüche 1 bis 7 verwendet.

13. Verfahren nach Anspruch 8,
**dadurch gekennzeichnet, daß** β-Citronellol zu Rosenoxid oxidiert wird.

14. Verwendung eines immobilisierten Photokatalysators nach einem der Ansprüche 1 bis 8 zur Behandlung von Abwasser.

15. Verwendung eines immobilisierten Photokatalysators nach einem der Ansprüche 1 bis 8 zur Oxidation von Thiolen, Sulfiden, Chlorphenolen, Phenolen, Dienen und/oder Steroiden.

16. Verwendung eines immobilisiertem Photokatalysator nach einem der Ansprüche 1 bis 8 zur Herstellung von Rosenoxid durch Photooxidation von β-Citronellol.

## Claims

1. Photocatalyst that comprises an active constituent immobilized on an ion exchange resin, whereby the photocatalytic active constituent is chosen from compounds of formulas I and II and combinations thereof 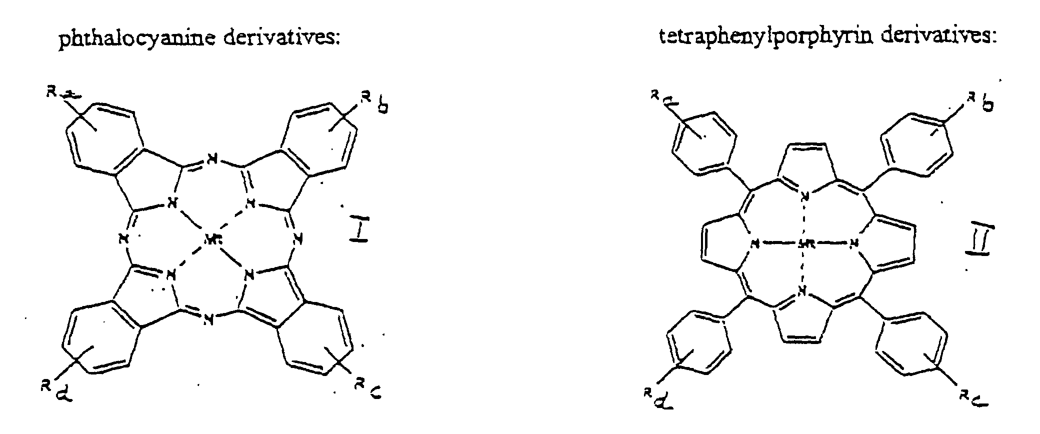 when Mt is a metal selected from aluminium, silicon, gallium and/or germanium and all remaining R are either each independently selected from anionic groups or from cationic groups and the indices a, b, c and d each independently indicate O or integers from 1 to 4, with the reservation that at least one of the indices a, b, c and d has a value other than 0.

2. Photocatalyst according to Claim 1,
**characterized in that** the carrier is an anion exchanger resin,

3. Photocatalyst according to Claim 1 or Claim 2,
**characterized in that** the substituent R is a halogen, OH, a phenolic group, -COOH, -SO₃H, a cabonic acid group or combinations thereof.

4. Photocatalyst according to Claim 1,
**characterized in that** the substituent R is an amino, trialkyl ammonium, trialkyl phosphonium, triaryl phosphonium, alkyl or aryl pyridinium or combinations thereof, whereby the alkyls can each have 1 to 18 carbon atoms and the aryls can have 6 to 12 carbon atoms.

5. Photocatalyst according to one of the preceding claims, **characterized in that** the active constituent is a metal-phthalocyanine and/or tetraphenylporphyrin derivative, in which R is a sulphonic acid or carboxy and a, b, c and d are each 1.

6. Photocatalyst according to one of the preceding claims, **characterized in that** the active constituent is phthalocyanine or tetraphenylporphyrin tetrasulphonate or a mixture thereof.

7. Photocatalyst according to one of the preceding claims,
**characterized in that** the metal of the phthalocyanine or tetraphenylporphyrin is aluminium(III), silicon(IV), gallium(III) and/or germanium(III).

8. Process for photo-oxidation using singlet oxygen as oxidant, the singlet oxygen is produced by bringing the compound to be oxidized into contact with a photocatalyst in accordance with one of Claims 1 to 7 with irradiation of light and in the presence of oxygen.

9. Process according to claim 8, **characterized in that** irradiation is with light of a wavelength of 400 to 800 nm.

10. Process according to Claim 8 or 9, **characterized in that** irradiation is with visible light or sunlight.

11. Process for photo-oxidation of biological material, **characterized in that** the biological material is brought into contact with an immobilized photocatalyst according to one of Claims 1 to 7 with irradiation of visible light and in the presence of oxygen.

12. Process according to one of Claims 8 to 11,
**characterized in that** a mixture of photocatalysts according to one of Claims 1 to 7 is used.

13. Process according to Claim 8,
**characterized in that** beta lemon grass oil is oxidized to rose oxide.

14. Use of an immobilized photocatalyst according to one of Claims 1 to 8 for the treatment of sewage.

15. Use of an immobilized photocatalyst according to one of Claims 1 to 8 for the oxidation of thiophens, sulphides, chlorophenols, phenols, dienes and/or steroids.

16. Use of an immobilized photocatalyst according to one of Claims 1 to 8 for the production of rose oxide by photo-oxidation of beta lemon grass oil.

## Revendications

1. Photocatalyseur, qui comprend un constituant photocatalytiquement actif immobilisé sur une résine échangeuse d'ions, où le constituant photocatalytiquement actif est choisi parmi les composés des formules I et II et leurs combinaisons 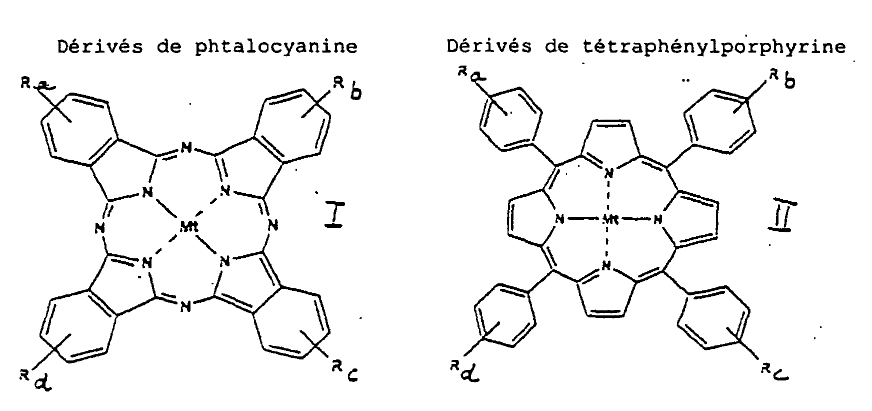 où Mt est un métal choisi parmi l'aluminium, le silicium, le gallium et/ou le germanium et tous les résidus R sont choisis chacun indépendamment parmi des radicaux anioniques ou des radicaux cationiques et les indices a, b, c et d représentent chacun indépendamment, 0 ou un nombre entier allant de 1 à 4, avec la réserve qu'au moins un des indices a, b, c et d a une autre signification que 0.

2. Photocatalyseur selon la revendication 1, **caractérisé en ce que** le support est une résine échangeuse d'anions.

3. Photocatalyseur selon l'une quelconque des revendications 1 et 2, **caractérisé en ce que** les substituants R sont un halogène, OH, un radical phénolique, -COOH, -SO₃H, un radical acide carboxylique ou une combinaison de ceux-ci.

4. Photocatalyseur selon la revendication 1, **caractérisé en ce que** les substituants R sont un résidu amino, trialkylammonium, trialkylphosphonium, triarylphosphonium, alkyl- ou arylpyridinium, ou des combinaisons de ceux-ci, où les résidus alkyle peuvent chacun présenter, 1 à 18 atomes de carbone et les résidus aryle peuvent présenter 6 à 12 atomes de carbone.

5. Photocatalyseur selon l'une quelconque des revendications précédentes, **caractérisé en ce que** le constituant actif est un dérivé métallique d'une phtalocyanine et/ou d'une tétraphénylporphyrine, où R est un résidu acide sulfonique ou un résidu carboxyle et a, b, c et d sont chacun, 1.

6. Photocatalyseur selon l'une quelconque des revendications précédentes, **caractérisé en ce que** le constituant actif est le tétrasulfonate de phtalocyanine ou de tétraphénylporphyrine ou un mélange de ceux-ci.

7. Photocatalyseur selon l'une quelconque des revendications précédentes, **caractérisé en ce que** le métal des phtalocyanines ou des tétraphényl-porphyrines est l'aluminium (III), le silicium (IV), le gallium (III) et/ou le germanium (IV).

8. Procédé de photooxydation en utilisant de l'oxygène singulet comme agent d'oxydation, où l'oxygène singulet est produit en ce que l'on met en contact le composé à oxyder sous rayonnement lumineux et en présence d'oxygène, avec un photocatalyseur selon l'une quelconque des revendications 1 à 7.

9. Procédé selon la revendication 8, **caractérisé en ce que** l'on irradie avec une lumière ayant une longueur d'onde allant de 400 à 800 nm.

10. Procédé selon l'une quelconque des revendications 8 et 9, **caractérisé en ce que** l'on irradie avec la lumière visible ou la lumière du soleil.

11. Procédé de photooxydation d'un matériau biologique, **caractérisé en ce que** l'on met en contact le matériau biologique avec un photocatalyseur immobilisé selon l'une quelconque des revendications 1 à 7, sous le rayonnement de la lumière visible et en présence d'oxygène.

12. Procédé selon l'une quelconque des revendications 8 à 11, **caractérisé en ce que** l'on utilise un mélange de photocatalyseurs selon l'une quelconque des revendications 1 à 7.

13. Procédé selon la revendication 8, **caractérisé en ce que** l'on oxyde le β-citronellol en oxyde de rose.

14. Utilisation d'un photocatalyseur immobilisé selon l'une quelconque des revendications 1 à 8, pour le traitement des eaux usées.

15. Utilisation d'un photocatalyseur immobilisé selon l'une quelconque des revendications 1 à 8, pour l'oxydation de thiols, de sulfures, de chlorophénols, de phénols, de diènes et/ou de stéroïdes.

16. Utilisation d'un photocatalyseur immobilisé selon l'une quelconque des revendications 1 à 8, pour la préparation d'oxyde de rose par photooxydation du β-citronellol.
